# EUROPÄISCHE PATENTSCHRIFT

(11) **EP 3 816 479 B1**
(45) Veröffentlichungstag und Bekanntmachung des Hinweises auf die Patenterteilung: **05.07.2023**
(21) Anmeldenummer: 19206020.0
(22) Anmeldetag: 29.10.2019
(51) Int. Cl.: F16H 25/22

(54) **PLANETENROLLENGEWINDETRIEB FÜR EINE SCHLUPFFREIE TELESKOPSPINDEL**
PLANETARY ROLLER SCREW DRIVE FOR A SLIP-FREE TELESCOPIC SPINDLE
VIS À ROULEAUX PLANÉTAIRES POUR UN BROCHE TÉLESCOPIQUE SANS GLISSEMENT

(43) Veröffentlichungstag der Anmeldung: 05.05.2021
(73) Patentinhaber: Maxon International AG, 6072 Sachseln (CH)
(72) Erfinder: von Lehmann, Ernst, 79286 Glottertal (DE); Mamier, Matthias, 79361 Sasbach (DE)
(74) Vertreter: Grünecker Patent- und Rechtsanwälte PartG mbB

(56) Entgegenhaltungen:
- EP-A1- 1 918 612
- EP-B1- 1 918 612
- DE-A1-102017 126 570
- US-A- 2 683 379

## Beschreibung

Die vorliegende Erfindung betrifft einen Planetenrollengewindetrieb nach dem Oberbegriff des unabhängigen Anspruchs 1.

Bei bekannten Planetenrollengewindetrieben, die als Teleskopspindel geeignet sind, wälzt ein Satz Planetenrollen zum einen auf einer zentralen Spindel und zum anderen auf einem Innengewinde einer Hohlradhülse ab. Aufgrund der von den Durchmesserverhältnissen abhängigen unterschiedlichen Drehzahlen der Spindel, der Planetenrollen bzw. der Hohlradhülse ergeben sich Steigungsdifferenzen, welche die gewünschte synchrone Hubbewegung erzeugen. Die zentrale Spindel hat dabei zwar die gleiche Anzahl an Gewindegängen wie die Planetenrollen aber im Vergleich zu den Planetenrollen einen kleineren Durchmesser. Das Gewinde der Hohlradhülse hat in der Regel die doppelte Anzahl an Gewindegängen wie die Planetenrollen. Die Wälzbewegung der Planetenrollen ist bei diesen bekannten Planetenrollengewindetrieben mit Schlupf verbunden, welcher nicht konstant ist, sondern von der Last abhängt. Sofern die Teleskopspindel als Aktuator eingesetzt werden soll, führt der Schlupf zu Ungenauigkeiten bei der Positionierung.

Planetenrollengewindetriebe sind beispielsweise aus DE 102015218621 A1 und EP 1 918 612 A1 bekannt. Die aus DE 102015218621 A1 und EP 1 918 612 A1 bekannten Planetenrollengewindetriebe sind jedoch nicht für eine Teleskopspindel geeignet, da eine relative axiale Verschiebung zwischen der Spindel und den Planetenrollen nicht möglich ist.

Ein Planetenrollengewindetrieb der gattungsgemäßen Art ist beispielsweise aus der DE 10 2017 126570 A1 bekannt. Dieser gattungsgemäße Planetenrollengewindetrieb weist eine Spindel mit einem Außengewinde, eine Schraubenmutter mit einem Innengewinde, sowie Planetenrollen auf, die zwischen der Spindel und der Schraubenmutter angeordnet sind und jeweils ein Außengewinde aufweisen, das sowohl mit dem Außengewinde der Spindel als auch mit dem Innengewinde der Schraubenmutter in Eingriff steht, wobei sowohl die Spindel als auch die Planetenrollen ferner jeweils eine Außenverzahnung aufweisen, und wobei die Außenverzahnung der Spindel mit der Außenverzahnung der Planetenrollen kämmt, wobei die Außenverzahnung der Spindel in das Außengewinde der Spindel und/oder die Außenverzahnung der Planetenrollen in das Außengewinde der Planetenrollen eingebracht sind, sodass Spindel und/oder Planetenrollen einen Überlagerungsabschnitt aufweisen, in dem sich das jeweilige Außengewinde und die jeweilige Außenverzahnung überlagern, derart dass eine relative axiale Verschiebung zwischen der Spindel und den Planetenrollen möglich ist, wobei der Planetenrollengewindetrieb ferner ein Hohlrad mit einer Innenverzahnung aufweist, wobei die Schraubenmutter drehfest in Bezug auf das Hohlrad ist, und wobei die Außenverzahnung der Planetenrollen mit der Innenverzahnung des Hohlrads kämmt. Der aus DE 10 2017 126570 A1 bekannte Planetenrollengewindetrieb ist jedoch nicht für eine Teleskopspindel geeignet.

Aufgabe der vorliegenden Erfindung ist es, einen Planetenrollengewindetrieb anzugeben, der für eine Teleskopspindel geeignet ist und bei welchem der oben angesprochene Schlupf reduziert ist.

Die Aufgabe wird gelöst durch die Merkmale des unabhängigen Anspruchs 1. Demnach liegt bei einem Planetenrollengewindetrieb nach dem Oberbegriff des unabhängigen Anspruchs 1 dann eine erfindungsgemäße Lösung der Aufgabe vor, wenn der Planetenrollengewindetrieb ein Gehäuse aufweist, an dem die Spindel drehbar gelagert ist, wobei das Hohlrad drehfest und axial verschieblich in Bezug auf das Gehäuse ist, wobei ferner die Schraubenmutter axial verschieblich in Bezug auf das Hohlrad gelagert ist, und wobei Durchmesserverhältnisse zwischen Spindel, Planetenrollen und Schraubenmutter derart gewählt sind, dass bei einer Rotation der Spindel sowohl eine relative axiale Verschiebung des Hohlrads in Bezug auf das Gehäuse als auch eine relative axiale Verschiebung der Schraubenmutter in Bezug auf das Hohlrad stattfindet..

Dadurch ist der Planetenrollengewindetrieb als kompakt bauende Teleskopspindel einsetzbar.

Durch den Eingriff der Außenverzahnungen von Spindel und Planetenrollen wird ein Schlupf zwischen Spindel und Planetenrollen unterbunden. Durch die Verzahnungen werden ferner die Drehzahlen der beteiligten Bauteile bestimmt. Die erfindungsgemäße Lösung erlaubt ferner eine kompakte Bauweise.

Spindel und Schraubenmutter sind vorzugsweise koaxial angeordnet. Weiter vorzugsweise weisen die Planetenrollen alle denselben Außendurchmesser auf. Besonders bevorzugt sind drei Planetenrollen vorgesehen, da sich bei dieser Ausführung ein Hubverhältnis von 1:2 einstellt. Alternativ können aber auch vier oder mehr Planetenrollen vorgesehen sein. Weitere vorteilhafte Ausführungsformen der vorliegenden Erfindung sind Gegenstand der Unteransprüche.

Gemäß einer bevorzugten Ausführungsform der vorliegenden Erfindung ist entweder die Außenverzahnung der Spindel in das Außengewinde der Spindel eingebracht, wobei sich die Außenverzahnung der Planetenrollen in einem axial außerhalb des Außengewindes der Planetenrollen bestehenden Abschnitt befindet, oder die Außenverzahnung der Planetenrollen ist in das Außengewinde der Planetenrollen eingebracht, wobei sich die Außenverzahnung der Spindel in einem axial außerhalb des Außengewindes der Spindel bestehenden Abschnitt befindet. Diese Ausführungsform lässt sich einfach herstellen und reduziert den Fertigungsaufwand. Das Bauteil, bei dem sich die Außenverzahnung und das Außengewinde nicht überlagern, kann einen einfachen Gewindeabschnitt mit einem axial daran anschließenden und einfach zu fertigenden Ritzel aufweisen.

Bevorzugt ist die Außenverzahnung der Spindel in das Außengewinde der Spindel eingebracht, wobei sich Außenverzahnung und Außengewinde der Planetenrollen nicht überlagern. Diese Ausführungsform lässt sich besonders einfach fertigen.

Gemäß einer besonders bevorzugten Ausführungsform ist die Außenverzahnung der Planetenrollen in das Außengewinde der Planetenrollen eingebracht, wobei sich die Außenverzahnung der Spindel in einem axial außerhalb des Außengewindes der Spindel bestehenden Abschnitt befindet. Um dabei sicherzustellen, dass die Außenverzahnung der Spindel trotz besonders kompakter Bauweise stets in das Außengewinde der Planetenrollen eingreift, kann gemäß einer vorteilhaften Ausführungsform vorgesehen sein, dass die Außenverzahnung der Spindel axial sowohl vor als auch nach dem Außengewinde der Spindel vorhanden ist. Vorteilhafterweise ist die Außenverzahnung der Spindel dabei durch zwei Ritzel gebildet, zwischen welchen sich das Außengewinde der Spindel erstreckt. Weiter vorzugsweise ist eines der beiden Ritzel ungefähr in der Mitte der Spindel angeordnet ist, wobei das andere der beiden Ritzel am distalen Ende der Spindel angeordnet ist. Ungefähr in der Mitte der Spindel bedeutet in diesem Zusammenhang in einem Bereich zwischen 35 % und 65 % der Gesamtlänge der Spindel, vorzugsweise in einem Bereich zwischen 40 % und 60 % der Gesamtlänge der Spindel. Unter dem distalen Ende wird in diesem Zusammenhang dasjenige Ende verstanden, welches in derjenigen Richtung, in welcher der Planetenrollengewindetrieb ausgefahren wird, vorne liegt. Das proximale Ende der Spindel ist demnach das gegenüberliegende Ende, welches zum feststehenden Ende des Planetenrollengewindetriebs weist. Von ganz besonderem Vorteil ist es, wenn ein proximaler Abschnitt der Spindel, in welchem weder Außengewinde noch Außenverzahnung der Spindel bestehen, durch einen Schaft gebildet ist, dessen Durchmesser vorzugsweise kleiner ist als ein Außendurchmesser des Außengewindes der Spindel und insbesondere kleiner als die Hälfte eines Außendurchmessers des Außengewindes der Spindel ist. Dadurch kann Gewicht und somit bewegte Masse eingespart werden.

In einer weiteren bevorzugten Ausführungsform der vorliegenden Erfindung weist der Überlagerungsabschnitt eine Länge auf, die zumindest 80 % der Länge des jeweiligen Außengewindes beträgt. Sofern der erfindungsgemäße Planetenrollengewindetrieb als Teleskopspindel zum Einsatz kommt, ergibt sich dadurch ein großer Verstellbereich. Besonders bevorzugt nimmt der Überlagerungsabschnitt die gesamte Länge des jeweiligen Außengewindes ein.

Gemäß einer weiteren bevorzugten Ausführungsform der vorliegenden Erfindung ist im Überlagerungsabschnitt eine in radialer Richtung gemessene Verzahnungshöhe der jeweiligen Außenverzahnung kleiner als eine ebenfalls in radialer Richtung gemessene Höhe der Gewindegänge des jeweiligen Außengewindes. Dadurch wird sichergestellt, dass die Gewindegänge der Außengewinde der Planetenrollen und der Spindel stets einwandfrei ineinandergreifen.

Alternativ oder zusätzlich kann vorteilhafter Weise vorgesehen sein, dass die Außenverzahnungen als Schrägverzahnungen ausgeführt sind. Dadurch wird auch die Überdeckung des Zahneingriffs zwischen den Planetenrollen und der Spindel erhöht.

Vorzugsweise handelt es sich bei dem Hohlrad um einen Hohlradring mit einer im Vergleich zur Schraubenmutter geringen axialen Ausdehnung.

Besonders bevorzugt ist es dabei, wenn das Hohlrad fest mit einer aus dem Gehäuse ausfahrbaren ersten Teleskophülse verbunden oder durch diese erste Teleskophülse gebildet ist, wobei die Schraubenmutter fest mit einer aus der ersten Teleskophülse ausfahrbaren zweiten Teleskophülse verbunden oder durch diese zweite Teleskophülse gebildet ist. Bei dieser Ausführungsform ergibt sich ein stabiler und gegen äußere Einflüsse geschützter Teleskopspindelantrieb.

Ausführungsbeispiele der vorliegenden Erfindung werden im Folgenden anhand von Zeichnungen näher erläutert.

Es zeigen:
- Figur 1:: einen als schlupffreie Teleskopspindel ausgeführten Planetenrollengewindetrieb in einer Schrägansicht im komplett eingefahrenen Zustand gemäß einem ersten Ausführungsbeispiel,
- Figur 2:: den Planetenrollengewindetrieb aus Figur 1 ebenfalls in Schrägansicht im komplett ausgefahrenen Zustand,
- Figur 3:: einen Längsschnitt des Planetenrollengewindetriebs aus den Figuren 1 und 2 im komplett eingefahrenen Zustand,
- Figur 4:: einen Längsschnitt des Planetenrollengewindetriebs aus den Figuren 1 bis 3 im komplett ausgefahrenen Zustand,
- Figur 5:: eine Detailansicht des Längsschnitts aus Figur 4 in einer perspektivischen Darstellung,
- Figur 6:: eine weitere Detailansicht des Längsschnitts aus Figur 4 in einer weiteren perspektivischen Darstellung, und
- Figur 7:: eine Abwandlung des Planetenrollengewindetriebs gemäß einem zweiten Ausführungsbeispiel in einem perspektivischen Längsschnitt.

Für die folgenden Ausführungen gilt, dass gleiche Teile durch gleiche Bezugszeichen bezeichnet sind. Sofern in einer Figur Bezugszeichen enthalten sind, auf die in der zugehörigen Figurenbeschreibung nicht näher eingegangen wird, so wird auf vorangehende oder nachfolgende Figurenbeschreibungen Bezug genommen.

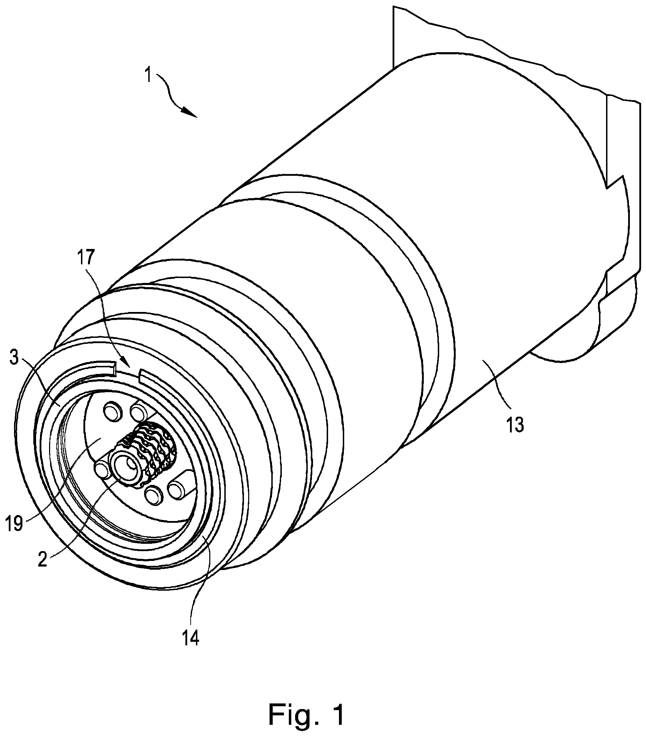

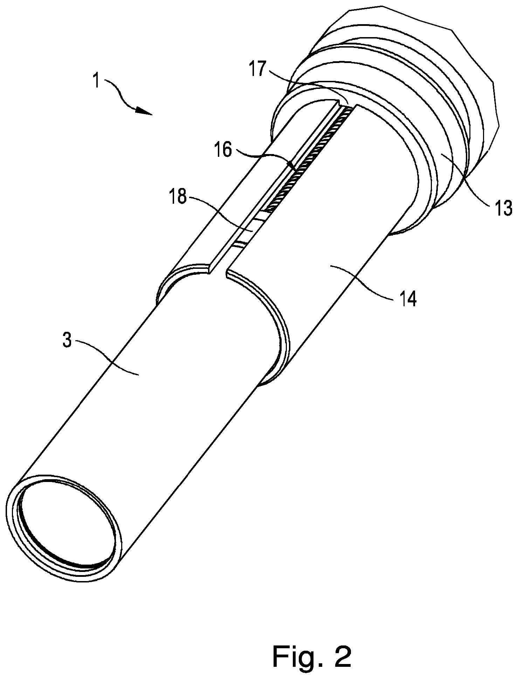

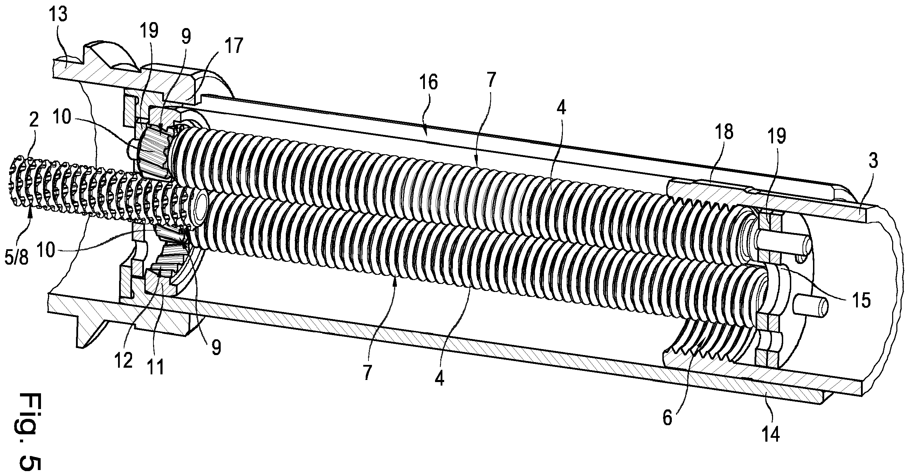

Figur 1 zeigt eine Schrägansicht eines erfindungsgemäßen Planetenrollengewindetriebs 1 im eingefahrenen Zustand. Figur 2 zeigt den Planetenrollengewindetrieb im ausgefahrenen Zustand. Der Planetenrollengewindetrieb weist ein äu ßeres Gehäuse 13, eine aus dem Gehäuse ausfahrbare erste Teleskophülse 14 sowie eine aus der ersten Teleskophülse ausfahrbare zweite Teleskophülse 3 auf. Beide Teleskophülsen sind drehfest in Bezug auf das Gehäuse 13 geführt. Wie insbesondere aus den Figuren 2 und 5 hervorgeht, weist die erste Teleskophülse 14 hierfür einen Längsschlitz 16 auf, in welchem ein entsprechender Führungszapfen 17 des Gehäuses 13 sowie ein entsprechender Führungszapfen 18 der zweiten Teleskophülse 3 aufgenommen und geführt sind.

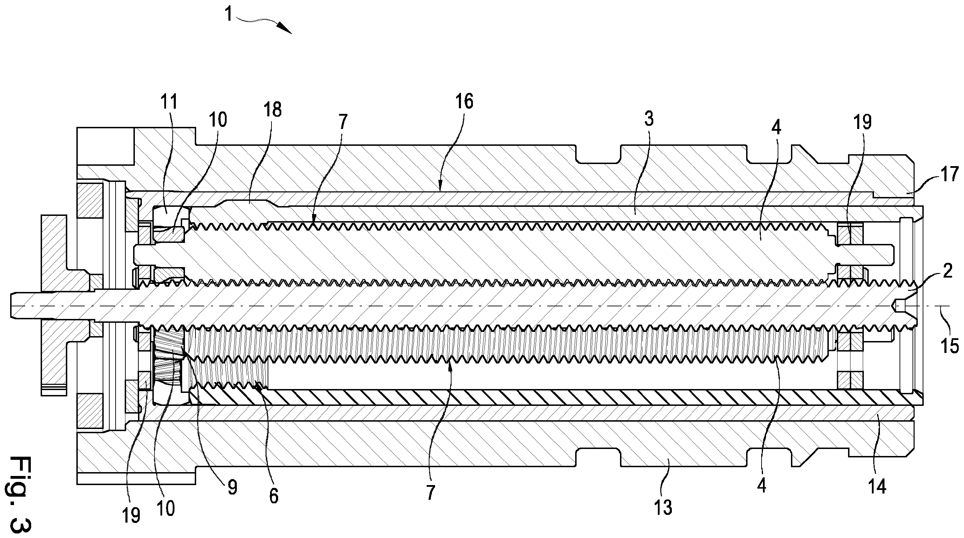

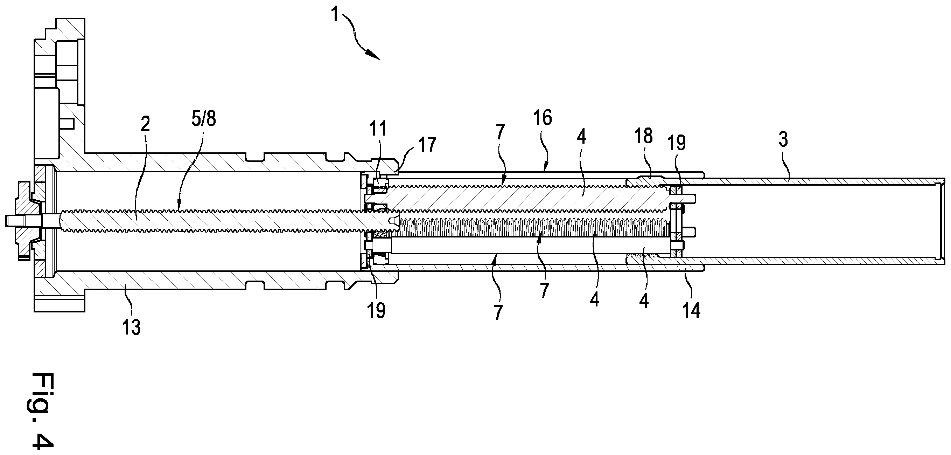

Die Figuren 3 und 4 zeigen jeweils Längsschnitte des erfindungsgemäßen Planetenrollengewindetriebs im eingefahrenen und im ausgefahrenen Zustand. Als Antrieb dient eine Spindel 2 mit einem Außengewinde 5, deren Achse 15 gleichzeitig die Achse des Gehäuses 13 sowie der beiden Teleskophülsen bildet. Insgesamt drei Planetenrollen 4 mit Außengewinde 7 sind derart zwischen der Spindel 2 und der als Schraubenmutter ausgeführten zweiten Teleskophülse 3 angeordnet, dass das Außengewinde 7 der Planetenrollen 4 sowohl mit dem Außengewinde 5 der Spindel als auch mit einem Innengewinde 6 der Schraubenmutter 3 in Eingriff steht. Die drei Planetenrollen 4 sind über zwei an den axialen Enden angeordnete Planetenrollenträger 19 drehbar in Bezug auf die Spindel 2, das Gehäuse 13 und die beiden Teleskophülsen 14 und 3 gelagert.

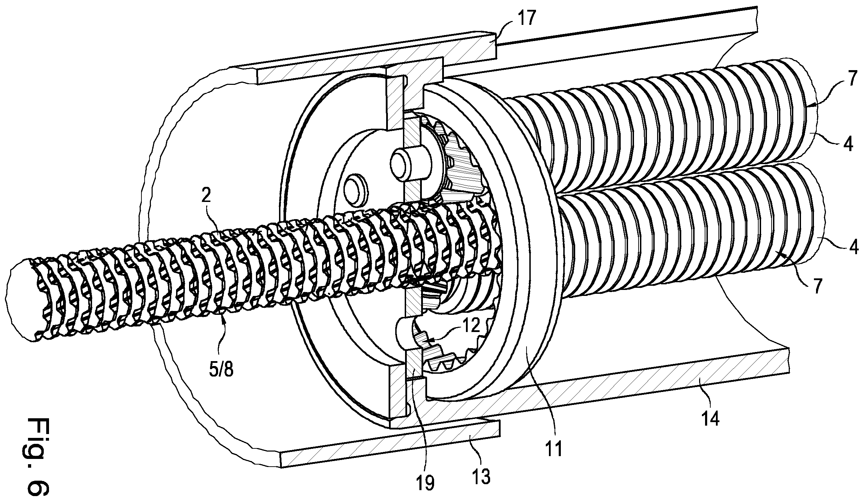

Wie insbesondere aus den Figuren 5 und 6 hervorgeht, weisen die Planetenrollen 4 nicht nur einen Gewindeabschnitt mit dem Außengewinde 7, sondern auch eine axial daran anschlie-ßende Au βenverzahnung 9 auf, die jeweils durch ein aufgesetztes Ritzel 10 gebildet wird. Die Ritzel 10 mit Außenverzahnung 9 kämmen sowohl mit einer Außenverzahnung 8 der Spindel 2 als auch mit einer Innenverzahnung 12 eines als Hohlradring ausgeführten Hohlrads 11, welches fest mit der ersten Teleskophülse 14 verbunden ist.

Die Außenverzahnung 8 der Spindel 2 ist bei diesem Ausführungsbeispiel in das Außengewinde 5 der Spindel 2 eingebracht, und zwar so, dass sich das Außengewinde 5 und die Au-βenverzahnung 8 auf der gesamten Länge des Außengewindes 5 überlagern. Dadurch wird eine relative axiale Verschiebung zwischen der Spindel 2 und den Planetenrollen 4 ermöglicht. Die Verzahnungshöhe der Außenverzahnung ist dabei kleiner als die Höhe der Gewindegänge des Außengewindes 5, sodass die Gewindegänge der Planetenrollen 4 und der Spindel 2 stets einwandfrei ineinandergreifen können. Die Außenverzahnungen 8 und 9 sowie die Innenverzahnung 12 sind als Schrägverzahnungen ausgeführt, um die Überdeckung des Zahneingriffs zu erhöhen.

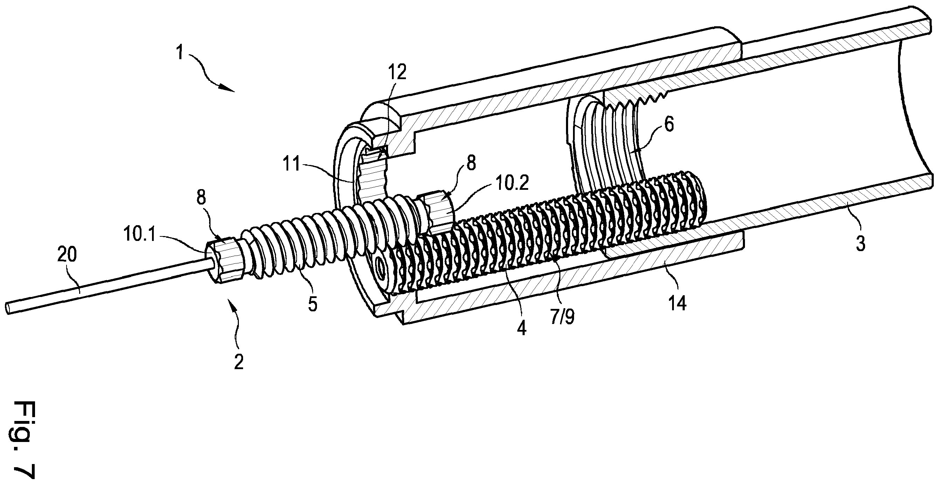

Figur 7 zeigt eine Abwandlung des erfindungsgemäßen Planetenrollengewindetriebs 1 aus den Figuren 1 bis 6 gemäß einem zweiten Ausführungsbeispiel. Der grundsätzliche Aufbau entspricht im Wesentlichen dem Planetenrollengewindetrieb 1 gemäß dem ersten Ausführungsbeispiel. Der Übersichtlichkeit halber wurde auf die Darstellung des Gehäuses und der Planetenrollenträger verzichtet. Ferner ist nur eine Planetenrolle 4 dargestellt. Der Planetenrollengewindetrieb 1 weist analog zum ersten Ausführungsbeispiel drei Planetenrollen auf, wobei die Anzahl der Planetenrollen selbstverständlich auch größer sein kann.

In Abweichung zum ersten Ausführungsbeispiel weisen die Planetenrollen 4 einen durchgehenden Gewindeabschnitt mit einem Außengewinde 7 auf, wobei die Außenverzahnung 9 der Planetenrollen 4 nicht durch ein zusätzlich vorgesehenes Ritzel gebildet wird sondern in das Außengewinde 7 der Planetenrollen 4 eingebracht ist. Die Außenverzahnung 9 besteht über die gesamte Länge des Außengewindes 7. Die Spindel 2 weist bei diesem Ausführungsbeispiel einen Gewindeabschnitt mit einem Außengewinde 5 auf, in welchen keine Außenverzahnung eingebracht ist. Anstatt dessen schließen sich axial vor und nach dem Gewindeabschnitt zwei Ritzel 10.1 und 10.2 an, durch welche die Außenverzahnung 8 der Spindel gebildet wird.

Beim zweiten Ausführungsbeispiel kann der Gewindeabschnitt der Spindel 2 bedeutend kürzer ausgeführt werden als beim ersten Ausführungsbeispiel. Der Gewindeabschnitt und die beiden Ritzel 10.1 und 10.2 erstrecken sich zusammen lediglich über etwas mehr als die Hälfte der Gesamtlänge der Spindel 2. Sie sind dabei zum distalen Ende der Spindel hin angeordnet. Der proximale Abschnitt der Spindel wird durch einen Schaft 20 mit einem im Vergleich zum Gewindeabschnitt deutlich kleineren Durchmesser gebildet, wodurch Gewicht und dadurch bewegte Masse eingespart werden kann. Im komplett eingefahrenen Zustand des Planetenrollengewindetriebs 1 befindet sich bei dieser Ausführungsvariante lediglich das etwa in der Mitte der Spindel angeordnete Ritzel 10.1 in Eingriff mit den Planetenrollen bzw. mit deren Außenverzahnung 9. Das am distalen Ende der Spindel 2 angeordnete Ritzel 10.2 befindet sich dabei nicht in Eingriff mit den Planetenrollen. Wird der Planetenrollengewindetrieb ausgefahren, so kommt zunächst das am distalen Ende der Spindel 2 angeordnete Ritzel 10.2 in Eingriff mit der Au βenverzahnung 9 der Planetenrollen 4. Im weiteren Verlauf kommt das Ritzel 10.1 außer Eingriff mit der Außenverzahnung 9 der Planetenrollen 4, sodass sich im komplett ausgefahrenen Zustand nur das Ritzel 10.2 in Eingriff mit der Außenverzahnung 9 der Planetenrollen 4 befindet.

### Bezugszeichenliste

- 1: Planetenrollengewindetrieb
- 2: Spindel
- 3: Schraubenmutter (Zweite Teleskophülse)
- 4: Planetenrolle
- 5: Außengewinde der Spindel
- 6: Innengewinde der Schraubenmutter
- 7: Außengewinde der Planetenrollen
- 8: Außenverzahnung der Spindel
- 9: Außenverzahnung der Planetenrollen
- 10: Ritzel
- 10.1: Ritzel
- 10.2: Ritzel
- 11: Hohlrad
- 12: Innenverzahnung des Hohlrads
- 13: Gehäuse
- 14: Erste Teleskophülse
- 15: Achse des Planetenrollengewindetriebs
- 16: Längsschlitz der ersten Teleskophülse
- 17: Führungszapfen des Gehäuses
- 18: Führungszapfen der zweiten Teleskophülse
- 19: Planetenrollenträger
- 20: Schaft

## Patentansprüche

1. Planetenrollengewindetrieb (1) mit
einer Spindel (2) mit einem Außengewinde (5),
einer Schraubenmutter (3) mit einem Innengewinde (6),
und mit Planetenrollen (4), die zwischen der Spindel (2) und der Schraubenmutter (3) angeordnet sind und jeweils ein Außengewinde (7) aufweisen, das sowohl mit dem Außengewinde (5) der Spindel (2) als auch mit dem Innengewinde (6) der Schraubenmutter (3) in Eingriff steht,
wobei sowohl die Spindel (2) als auch die Planetenrollen (4) ferner jeweils eine Außenverzahnung (8, 9) aufweisen, und wobei die Außenverzahnung (8) der Spindel (2) mit der Außenverzahnung (9) der Planetenrollen (4) kämmt,
wobei die Außenverzahnung (8) der Spindel (2) in das Außengewinde (5) der Spindel (2) und/oder die Außenverzahnung (9) der Planetenrollen (4) in das Außengewinde (7) der Planetenrollen (4) eingebracht sind, sodass Spindel (2) und/oder Planetenrollen (4) einen Überlagerungsabschnitt aufweisen, in dem sich das jeweilige Außengewinde (5, 7) und die jeweilige Außenverzahnung (8, 9) überlagern, derart dass eine relative axiale Verschiebung zwischen der Spindel (2) und den Planetenrollen (4) möglich ist, wobei der Planetenrollengewindetrieb (1) ferner ein Hohlrad (11) mit einer Innenverzahnung (12) aufweist, wobei die Schraubenmutter (3) drehfest in Bezug auf das Hohlrad (11) ist, und wobei die Außenverzahnung (9) der Planetenrollen (4) mit der Innenverzahnung (12) des Hohlrads (11) kämmt, und wobei der Planetenrollengewindetrieb (1) ein Gehäuse (13) aufweist, an dem die Spindel (2) drehbar gelagert ist,
**dadurch gekennzeichnet, dass** das Hohlrad (11) drehfest und axial verschieblich in Bezug auf das Gehäuse (13) ist, wobei ferner die Schraubenmutter (3) axial verschieblich in Bezug auf das Hohlrad (11) gelagert ist, und wobei Durchmesserverhältnisse zwischen Spindel (2), Planetenrollen (4) und Schraubenmutter (3) derart gewählt sind, dass bei einer Rotation der Spindel (2) sowohl eine relative axiale Verschiebung des Hohlrads (11) in Bezug auf das Gehäuse (13) als auch eine relative axiale Verschiebung der Schraubenmutter (3) in Bezug auf das Hohlrad (11) stattfindet.

2. Planetenrollengewindetrieb (1) nach Anspruch 1, **dadurch gekennzeichnet, dass** entweder die Außenverzahnung (8) der Spindel (2) in das Außengewinde (5) der Spindel (2) eingebracht ist, wobei sich die Außenverzahnung (9) der Planetenrollen (4) in einem axial außerhalb des Außengewindes (7) der Planetenrollen (4) bestehenden Abschnitt befindet, oder die Außenverzahnung (9) der Planetenrollen (4) in das Außengewinde (7) der Planetenrollen (4) eingebracht ist, wobei sich die Außenverzahnung (8) der Spindel (2) in einem axial außerhalb des Außengewindes (5) der Spindel (2) bestehenden Abschnitt befindet.

3. Planetenrollengewindetrieb (1) nach Anspruch 2, **dadurch gekennzeichnet, dass** die Außenverzahnung (8) der Spindel (2) in das Außengewinde (5) der Spindel (2) eingebracht ist.

4. Planetenrollengewindetrieb (1) nach Anspruch 2, **dadurch gekennzeichnet, dass** die Außenverzahnung (9) der Planetenrollen (4) in das Außengewinde (7) der Planetenrollen (4) eingebracht ist, wobei sich die Außenverzahnung (8) der Spindel (2) in einem axial außerhalb des Außengewindes (5) der Spindel (2) bestehenden Abschnitt befindet.

5. Planetenrollengewindetrieb (1) nach Anspruch 4, **dadurch gekennzeichnet, dass** die Außenverzahnung (8) der Spindel (2) axial sowohl vor als auch nach dem Außengewinde (5) der Spindel (2) vorhanden ist.

6. Planetenrollengewindetrieb (1) nach Anspruch 5, **dadurch gekennzeichnet, dass** die Außenverzahnung (8) der Spindel (2) durch zwei Ritzel (10.1, 10.2) gebildet ist, zwischen welchen sich das Außengewinde (5) der Spindel (2) erstreckt.

7. Planetenrollengewindetrieb (1) nach Anspruch 6, **dadurch gekennzeichnet, dass** eines der beiden Ritzel (10.1, 10.2) ungefähr in der Mitte der Spindel (2) angeordnet ist, wobei das andere der beiden Ritzel (10.1, 10.2) am distalen Ende der Spindel (2) angeordnet ist.

8. Planetenrollengewindetrieb (1) nach einem der Ansprüche 4 bis 7, **dadurch gekennzeichnet, dass** ein proximaler Abschnitt der Spindel (2), in welchem weder Außengewinde (5) noch Außenverzahnung (8) der Spindel (2) bestehen, durch einen Schaft (20) gebildet ist, dessen Durchmesser kleiner ist als ein Außendurchmesser des Außengewindes (5) der Spindel (2) und insbesondere kleiner als die Hälfte eines Außendurchmessers des Außengewindes (5) der Spindel (2) ist.

9. Planetenrollengewindetrieb (1) nach einem der Ansprüche 1 bis 8, **dadurch gekennzeichnet, dass** der Überlagerungsabschnitt eine Länge aufweist, die zumindest 80 % der Länge des jeweiligen Außengewindes (5, 7) beträgt.

10. Planetenrollengewindetrieb (1) nach Anspruch 9, **dadurch gekennzeichnet, dass** der Überlagerungsabschnitt die gesamte Länge des jeweiligen Außengewindes (5, 7) einnimmt.

11. Planetenrollengewindetrieb (1) nach einem der Ansprüche 1 bis 10, **dadurch gekennzeichnet, dass** im Überlagerungsabschnitt eine Verzahnungshöhe der jeweiligen Außenverzahnung (8, 9) kleiner ist als eine Höhe der Gewindegänge des jeweiligen Außengewindes (5, 7).

12. Planetenrollengewindetrieb (1) nach einem der Ansprüche 1 bis 11, **dadurch gekennzeichnet, dass** die Außenverzahnungen (8, 9) als Schrägverzahnungen ausgeführt sind.

13. Planetenrollengewindetrieb (1) nach einem der Ansprüche 1 bis 12, **dadurch gekennzeichnet, dass** das Hohlrad (11) fest mit einer aus dem Gehäuse (13) ausfahrbaren ersten Teleskophülse (14) verbunden oder durch diese erste Teleskophülse (14) gebildet ist, wobei die Schraubenmutter (3) fest mit einer aus der ersten Teleskophülse (14) ausfahrbaren zweiten Teleskophülse verbunden oder durch diese zweite Teleskophülse gebildet ist.

## Claims

1. A planetary roller screw (1) comprising:
a spindle (2) having a male thread (5),
a nut (3) having a female thread (6),
and planetary rollers (4) arranged between the spindle (2) and the nut (3) and each including a male thread (7) which engages both the male thread (5) of the spindle (2) and the female thread (6) of the nut (3),
wherein the spindle (2) and the planetary rollers (4) each include external toothing (8, 9), the external toothing (8) of the spindle (2) meshing with the external toothing (9) of the planetary rollers (4),
wherein the external toothing (8) of the spindle (2) is engaged in the male thread (5) of the spindle (2) and/or the external toothing (9) of the planetary rollers (4) is engaged in the male thread (7) of the planetary rollers (4), so that the spindle (2) and/or the planetary rollers (4) include an overlapping section in which the respective male thread (5, 7) and the respective external toothing (8, 9) overlap such that a relative axial displacement between the spindle (2) and the planetary rollers (4) is enabled, wherein the planetary roller screw (1) further includes a ring gear (11) having internal toothing (12), wherein the nut (3) is non-rotatable with respect to the ring gear (11), and wherein the external toothing (9) of the planetary rollers (4) meshes with the internal toothing (12) of the ring gear (11), and wherein the planetary roller screw (1) comprises a housing (13) on which the spindle (2) is rotatably supported,
**characterized in that**
the ring gear (11) is non rotatable and axially displaceable with respect to the housing (13), wherein furthermore the nut (3) is supported axially displaceably with respect to the ring gear (11), and wherein diameter ratios between spindle (2), planetary rollers (4) and nut (3) are selected such that, upon rotation of the spindle (2), both a relative axial displacement of the ring gear (11) with respect to the housing (13) and a relative axial displacement of the nut (3) with respect to the ring gear (11) take place.

2. The planetary roller screw (1) according to claim 1, **characterized in that** either the external toothing (8) of the spindle (2) is engaged in the male thread (5) of the spindle (2), wherein the external toothing (9) of the planetary rollers (4) is located in a section axially outside the male thread (7) of the planetary rollers (4), or the external toothing (9) of the planetary rollers (4) is engaged in the male thread (7) of the planetary rollers (4), wherein the external toothing (8) of the spindle (2) is located in a section axially outside the male thread (5) of the spindle (2).

3. The planetary roller screw (1) according to claim 2, **characterized in that** the external toothing (8) of the spindle (2) is engaged in the male thread (5) of the spindle (2).

4. The planetary roller screw (1) according to claim 2, **characterized in that** the external toothing (9) of the planetary rollers (4) is engaged in the male thread (7) of the planetary rollers (4) wherein the external toothing (8) of the spindle (2) is located in a section axially outside the male thread (5) of the spindle (2).

5. The planetary roller screw (1) according to claim 4, **characterized in that** the external toothing (8) of the spindle (2) is present axially both in front of and at the back of the male thread (5) of the spindle (2).

6. The planetary roller screw (1) according to claim 5, **characterized in that** the external toothing (8) of the spindle (2) is formed by two pinions (10.1, 10.2) wherein the male thread (5) of the spindle (2) extends therebetween.

7. The planetary roller screw (1) according to claim 6, **characterized in that** one of the two pinions (10.1, 10.2) is arranged approximately in the center of the spindle (2) wherein the other one of the two pinions (10.1, 10.2) is arranged at the distal end of the spindle (2).

8. The planetary roller screw (1) according to any one of claims 4 to 7, **characterized in that** a proximal portion of the spindle (2), in which neither male thread (5) nor external toothing (8) of the spindle (2) exists, is formed by a shaft (20), the diameter of which is smaller than an external diameter of the male thread (5) of the spindle (2) and in particular smaller than half of an external diameter of the male thread (5) of the spindle (2).

9. The planetary roller screw (1) according to any one of claims 1 to 8, **characterized in that** the overlapping section has a length of at least 80% of the length of the respective male thread (5, 7).

10. The planetary roller screw (1) according to claim 9, **characterized in that** the overlapping section occupies the entire length of the respective male thread (5, 7).

11. The planetary roller screw (1) according to any one of claims 1 to 10, **characterized in that** in the overlapping section, a height of the teeth of the respective external toothing (8, 9) is smaller than a height of the threads of the respective male thread (5, 7).

12. The planetary roller screw (1) according to any one of claims 1 to 11, **characterized in that** the external toothing (8) is configured as helical teeth.

13. The planetary roller screw (1) according to any one of claims 1 to 12, **characterized in that** the ring gear (11) is fixedly connected to a first telescopic sleeve (14) extendable from the housing (13) or is formed by this first telescopic sleeve (14), wherein the nut (3) is fixedly connected to a second telescopic sleeve extendable from the first telescopic sleeve (14) or is formed by this second telescopic sleeve.

## Revendications

1. Transmission par vis à rouleaux planétaires (1) comprenant
une vis (2) munie d'un filetage extérieur (5),
un écrou (3) muni d'un filetage intérieur (6),
et comprenant des rouleaux planétaires (4) agencés entre la vis (2) et l'écrou (3) et présentant respectivement un filetage extérieur (7) en prise aussi bien avec le filetage extérieur (5) de la vis (2) qu'avec le filetage intérieur (6) de l'écrou (3),
dans laquelle aussi bien la vis (2) que les rouleaux planétaires (4) présentent en outre respectivement une denture extérieure (8, 9), et dans laquelle la denture extérieure (8) de la vis (2) engrène avec la denture extérieure (9) des rouleaux planétaires (4),
dans laquelle la denture extérieure (8) de la vis (2) est introduite dans le filetage extérieur (5) de la vis (2) et/ou la denture extérieure (9) des rouleaux planétaires (4) est introduite dans le filetage extérieur (7) des rouleaux planétaires (4), de sorte que la vis (2) et/ou les rouleaux planétaires (4) présente(nt) une section de superposition au sein de laquelle le filetage extérieur (5, 7) respectif et la denture extérieure (8, 9) respective se superposent, de telle manière qu'un déplacement axial relatif entre la vis (2) et les rouleaux planétaires (4) est possible, dans laquelle la transmission par vis à rouleaux planétaires (1) présente en outre une roue creuse (11) avec une denture intérieure (12), dans laquelle l'écrou (3) est solidaire en rotation par rapport à la roue creuse (11), et dans laquelle la denture extérieure (9) des rouleaux planétaires (4) engrène avec la denture intérieure (12) de la roue creuse (11), et dans laquelle la transmission par vis à rouleaux planétaires (1) présente une cage (13) au niveau de laquelle la transmission par vis à rouleaux planétaires (1) est montée de manière rotative,
**caractérisée en ce que**
la roue creuse (11) est solidaire en rotation et mobile axialement par rapport à la cage (13), dans laquelle l'écrou (3) est en outre monté de manière mobile axialement par rapport à la roue creuse (11), et dans laquelle des rapports de diamètre entre la vis (2), les rouleaux planétaires (4) et l'écrou (3) sont choisis de telle manière que, lors d'une rotation de la vis (2), il se produit aussi bien un déplacement axial relatif de la roue creuse (11) par rapport à la cage (13) qu'un déplacement axial relatif de l'écrou (3) par rapport à la roue creuse (11).

2. Transmission par vis à rouleaux planétaires (1) selon la revendication 1, **caractérisée en ce que** soit la denture extérieure (8) de la vis (2) est introduite dans le filetage extérieur (5) de la vis (2), dans laquelle la denture extérieure (9) des rouleaux planétaires (4) se trouve dans une section constituée de manière axiale à l'extérieur du filetage extérieur (7) des rouleaux planétaires (4), soit la denture extérieure (9) des rouleaux planétaires (4) est introduite dans le filetage extérieur (7) des rouleaux planétaires (4), dans laquelle la denture extérieure (8) de la vis (2) se trouve dans une section constituée de manière axiale à l'extérieur du filetage extérieur (5) de la vis (2).

3. Transmission par vis à rouleaux planétaires (1) selon la revendication 2, **caractérisée en ce que** la denture extérieure (8) de la vis (2) est introduite dans le filetage extérieur (5) de la vis (2).

4. Transmission par vis à rouleaux planétaires (1) selon la revendication 2, **caractérisée en ce que** la denture extérieure (9) des rouleaux planétaires (4) est introduite dans le filetage extérieur (7) des rouleaux planétaires (4), dans laquelle la denture extérieure (8) de la vis (2) se trouve dans une section située de manière axiale à l'extérieur du filetage extérieur (5) de la vis (2).

5. Transmission par vis à rouleaux planétaires (1) selon la revendication 4, **caractérisée en ce que** la denture extérieure (8) de la vis (2) est présente de manière axiale aussi bien avant qu'après le filetage extérieur (5) de la vis (2).

6. Transmission par vis à rouleaux planétaires (1) selon la revendication 5, **caractérisée en ce que** la denture extérieure (8) de la vis (2) est formée de deux pignons (10.1, 10.2) entre lesquels s'étend le filetage extérieur (5) de la vis (2).

7. Transmission par vis à rouleaux planétaires (1) selon la revendication 6, **caractérisée en ce que** l'un des deux pignons (10.1, 10.2) est agencé approximativement au milieu de la vis (2), dans laquelle l'autre des deux pignons (10.1, 10.2) est agencé à l'extrémité distale de la vis (2).

8. Transmission par vis à rouleaux planétaires (1) selon l'une quelconque des revendications 4 à 7, **caractérisée en ce qu'**une section proximale de la vis (2), au sein de laquelle il n'existe ni filetage extérieur (5) ni denture extérieure (8) de la vis (2), est formée d'une tige (20) dont le diamètre est inférieur à un diamètre extérieur du filetage extérieur (5) de la vis (2) et est en particulier inférieur à la moitié d'un diamètre extérieur du filetage extérieur (5) de la vis (2).

9. Transmission par vis à rouleaux planétaires (1) selon l'une quelconque des revendications 1 à 8, **caractérisée en ce que** la section de superposition présente une longueur qui représente au moins 80 % de la longueur du filetage extérieur (5, 7) respectif.

10. Transmission par vis à rouleaux planétaires (1) selon la revendication 9, **caractérisée en ce que** la section de superposition occupe toute la longueur du filetage extérieur (5, 7) respectif.

11. Transmission par vis à rouleaux planétaires (1) selon l'une quelconque des revendications 1 à 10, **caractérisée en ce que**, au sein de la section de superposition, une hauteur de denture de la denture extérieure (8, 9) respective est inférieure à une hauteur des filetages du filetage extérieur (5, 7) respectif.

12. Transmission par vis à rouleaux planétaires (1) selon l'une quelconque des revendications 1 à 11, **caractérisée en ce que** les dentures extérieures (8, 9) sont réalisées sous forme de dentures obliques.

13. Transmission par vis à rouleaux planétaires (1) selon l'une quelconque des revendications 1 à 12, **caractérisée en ce que** la roue creuse (11) est reliée de manière fixe à une première douille télescopique (14) pouvant être sortie de la cage (13) ou est formée de ladite première douille télescopique (14), dans laquelle l'écrou (3) est relié de manière fixe à une seconde douille télescopique pouvant être sortie de la première douille télescopique (14) ou est formé de ladite seconde douille télescopique.
